# EUROPÄISCHE PATENTANMELDUNG

(11) **EP 0 926 128 A1**
(43) Veröffentlichungstag der Anmeldung: **30.06.1999**
(21) Anmeldenummer: 98121358.0
(22) Anmeldetag: 10.11.1998
(51) Int. Cl.: C07C 67/31, C07C 69/708, C07C 67/03

(54) **Verfahren zur Herstellung von Methoxyessigsäureestern**

(30) Priorität: 15.12.1997 DE 19755599
(71) Anmelder: HÜLS AKTIENGESELLSCHAFT, 45764 Marl (DE)
(72) Erfinder: Kleemiss, Wolfgang, Dr., 45721 Haltern (DE); Steffen, Klaus-Dieter, Dr., 53773 Hennef (DE); Feld, Marcel, Dr., 51147 Köln (DE)

(57) **Zusammenfassung**

Die Erfindung betrifft ein Verfahren zur Herstellung von Methoxyessigsäureestern, bei dem ein Chloressigsäureester mit einem Alkalimethylat zum Methoxyessigsäureester umgesetzt wird.

## Beschreibung

Die Erfindung betrifft ein Verfahren zur Herstellung von Methoxyessigsäureestern durch Umsetzung von Chloressigsäureestern mit einem Alkalimethylat.

Methoxyessigsäureester sind wertvolle Zwischenprodukte, die z.B. zur kinetischen Racematspaltung von chiralen Aminen Verwendung finden (F.Balkenhohl, K.Ditrich, B.Hauer, W.Ladner, J.prakt.Chem. 339 (1997), 381-384; DE-OS 195 23 151). An die Reinheit des Methoxyessigsäureesters werden dabei hohe Anforderungen gestellt, da die zur Racematspaltung verwendete Lipase durch Verunreinigungen leicht inhibiert wird. Besonders kritisch ist dabei ein Gehalt an Chloressigsäureester und an Wasser.

Methoxyessigsäureester können prinzipiell durch Veresterung von Methoxyessigsäure hergestellt werden (Paloma, Jakkola, Chem.Ber. 67 (1967),954; G.G.Smith et al., J.Org.Chem. 42 (1977), 44-47; Kumar, K.Amal, Chattopadhay, K.Tapas, Tetrahedron Lett. 28 (1987), 3713-3714). Die hierbei erzielten Ausbeuten von bis zu 60 % sind jedoch nicht zufriedenstellend.

Zudem ist Methoxyessigsäure nur schwer zugänglich. Sie kann z.B. durch Salpetersäure- oder elektrochemische Oxidation von Ethylenglykolmonomethylether hergestellt werden (DE-OS 32 09 622; EP 0 048 396; DE-OS 36 20 013). Bei der elektrochemischen Oxidation entsteht primär das Natriumsalz, das mit einer anderen Säure in die freie Methoxyessigsäure überführt werden muß. Dabei entsteht als Zwangsanfall das Natriumsalz dieser anderen Säure, das entsorgt werden muß. Bei der Salpetersäure-Oxidation muß ein 2,5-facher stöchiometrischer Überschuß an Salpetersäure angewandt werden. Schließlich kann man Ethylenglykolmonomethylether auch durch katalytische Oxidation, mit Luftsauerstoff zur Methoxyessigsäure oxidieren (DE 293 61 23), die dabei jedoch als stark verdünnte wäßrige Lösung anfällt und energieaufwendig zum wasserfreien Produkt aufdestilliert werden muß. Alle von Ethylenglykolmonomethylether ausgehende Oxidationsverfahren haben außerdem den Nachteil, daß im Reaktionsprodukt neben Reaktionsnebenprodukten auch noch Ausgangsstoff vorhanden ist. Ethylenglykolmonomethylether steht jedoch im Verdacht, fruchtschädigend zu sein, so daß im Umgang mit derart verunreinigter Methoxyessigsäure oder den entsprechenden Estern besondere Vorsicht geboten ist.

Methoxyessigsäuremethylester ist auch durch Carbonylierung von Methanol (US 4 482 735) oder von Formaldehyddimethylacetal (EP 0 071 920) zugänglich. Die Selektivitäten und damit die Ausbeuten der jeweiligen Carbonylierungsreaktionen sind jedoch gering, so daß die Verfahren für eine wirtschaftliche Produktion von Methoxyessigsäureestern gänzlich ungeeignet sind.

Ein weiteres, gut zugängliches Ausgangsmaterial für die Herstellung von Methoxyessigsäureestern ist Chloressigsäure. Man kann deren Alkalisalze oder das Ammoniumsalz mit Alkalimethylat in das Alkali- oder Ammoniumsalz der Methoxyessigsäure überführen (DE-OS 29 48 200). Dieses wird durch Protonierung in die Methoxyessigsäure umgewandelt, die dann, wie zuvor beschrieben, verestert werden muß. Dieses Verfahren ist schon wegen der unbefriedigenden Ausbeuten der Veresterungsstufe unwirtschaftlich und zudem mit großem Salzanfall verbunden.

Eine wesentliche Aufgabe der Erfindung besteht darin, ein von gut zugänglichen, billigen Ausgangsmaterialien ausgehendes, wirtschaftliches Verfahren bereitzustellen, das in einfacher Weise Methoxyessigsäureester in guten Ausbeuten und mit hoher Reinheit ergibt.

Es wurde nun gefunden, daß diese Aufgabe gelöst wird durch ein Verfahren zur Herstellung von Methoxyessigsäureestern, bei dem ein Chloressigsäureester mit einem Alkalimethylat zum Methoxyessigsäureester umgesetzt wird. Dieser kann gewünschtenfalls nach seiner Isolierung aus dem Reaktionsgemisch oder direkt im Reaktionsgemisch umgeestert werden. Diese Umesterung gelingt besonders einfach, wenn der Methoxyessigsäureester mit einem höheren Alkohol umgeestert wird. Auf diese Weise läßt sich z.B. der Methoxyessigsäuremethylester durch Umsetzung mit einem höheren Alkohol unter Abdestillieren von Methanol in Methoxyessigsäureester beliebiger höherer Alkohole umwandeln.

Nach dem erfindungsgemäßen Verfahren werden auch mit überschüssigem Alkalialkoholat gute Ausbeuten erzielt, die je nach Ester bis zu 90 % betragen können. Das ist überraschend, denn es ist bekannt, daß der Methoxyessigsäuremethylester in Gegenwart von Alkalimethylat ein Kondensationsprodukt ergibt (H.Adkins, R.M.Elofson, A.G.Rossow, C.C.Robinson; J.Am.Soc 71, 3622 [1949]).

Weiterhin ist überraschend, daß Methoxyessigsäureester direkt im Reaktionsgemisch der Reaktion von Chloressigsäureester mit überschüssigem Alkalimethylat, in dem noch Alkalimethylat neben dem Alkalichlorid vorliegt, das bei der Reaktion in stöchiometrischen Mengen entsteht, unter Zusatz eines höheren Alkohols und unter Abdestillieren des niederen Alkohols direkt zum Methoxyessigsäureester des höheren Alkohols umgeestert werden kann und dieser, gegebenenfalls nach vorheriger Abtrennung des Alkalichlorids, durch Destillation in hohen Ausbeuten gewonnen werden kann. Üblicherweise werden nämlich als Umesterungskatalysatoren Lewissäuren, wie Alkoxytitanate, verwendet (D.Seebach et al., Synthesis 1982, S.138), während die Verwendung der stark basischen Alkalialkoholate für diesen Zweck ausgesprochen unüblich ist.

Überraschend ist ferner der niedrige Chlorgehalt der direkt hergestellten Methoxessigsäureester sowie der durch Umesterung daraus hergestellten höheren Methoxyessigsäureester, der lediglich 5 bis 10 ppm beträgt. Die Ester eignen sich daher auch und besonders für die erwähnte kinetische enzymatische Racematspaltung von chiralen Aminen.

Bevorzugte Chloressigsäureester sind diejenigen, die sich von Alkanolen mit 1 bis 6 oder Alkoxyalkanolen mit 3 bis 6 Kohlenstoffatomen ableiten. Beispielhaft seien die Chloressigsäureester des Methanols, Ethanols, 1- oder 2-Propanols, 1- oder 2-Butanols, 2-Methyl-1-propanols, 1-Hexanols und 3-Oxa-1-pentanols (oder Ethylglykols) genannt. Besonders bevorzugt werden der Methyl- und der Ethylester.

Das bevorzugte Alkalimethylat ist neben Kaliummethylat das wohlfeile Natriummethylat. Das Alkalimethylat wird im allgemeinen in einer Menge von 1 bis 2, vorteilhaft von 1 bis 1, 5 und insbesondere von 1 bis 1,2 Molen je Mol Chloressigsäureester angewandt.

Das erfindungsgemäße Verfahren wird zweckmäßig bei einer Temperatur von 30 bis 100°C, vorteilhaft von 60 bis 80°C durchgeführt. Das Reaktionsgemisch kann durch Destillation aufgearbeitet werden, gegebenenfalls nach vorheriger Neutralisation des überschüssigen Alkalimethylats, z.B. mittels einer Mineralsäure, wie Schwefelsäure.

Der Methoxyessigsäureester kann nach seiner Abtrennung durch Destillation oder, wie erwähnt, überraschenderweise mit gutem Erfolg auch im Reaktionsgemisch durch Umesterung mit einem höheren Alkohol unter Abdestillieren des niederen Alkohols zum Methoxyessigsäureester des höheren Alkohols umgesetzt werden. Geeignete höhere Alkohole sind vorzugsweise Alkanole mit 2 bis 10 Kohlenstoffatomen und Alkoxyalkanole mit 4 bis 10 Kohlenstoffatomen. Im einzelnen seien beispielsweise 2-Ethylhexanol, n-Octanol, die Nonanole und die Decanole, jeweils für sich allein oder als Isomerengemische; sowie 2-Ethoxyethanol und 2-n-Butoxyethanol genannt. Die Umesterung wird zweckmäßig bei Temperaturen von 60 bis 250°C so durchgeführt, daß der niedere Alkohol abdestilliert.

Als Katalysatoren für die Umesterung kommen neben den erwähnten Alkalimethylaten u.a. Alkalialkoholate von Alkoholen mit 1 bis 8 Kohlenstoffatomen sowie Lewis-saure Katalysatoren, wie Alkoxytitanate oder -stannate, Eisen(III)-chlorid oder Zinn(II)-chlorid, oder Broenstedt-Säuren, wie 4-Toluylsulfonsäure, in Betracht.

Sowohl die Herstellung der Methoxyessigsäureester aus Chloressigsäureestern als auch gegebenenfalls die Umesterung können unter Umgebungsdruck, erhöhten oder erniedrigten Druck durchgeführt werden. Vorteilhaft arbeitet man bei einem Druck von 0,8 bis 3 bar, vorteilhaft von 0,9 bis 1,5 bar. Bei der Umesterung ist es zweckmäßig, Druck und Temperatur so zu korrelieren, daß der niedere Alkohol abdestilliert.

Zur Aufarbeitung der Reaktionsgemische kann man zunächst das entstandene Alkalichorid abtrennen, z.B. durch Filtrieren oder durch Absaugen der flüssige Phase vom Alkalichlorid, und den Methoxyessigsäureester durch Fraktionieren der flüssigen Phase gewinnen, wobei etwaiges überschüssiges Alkalialkoholat im Destillationsrückstand verbleibt. Alternativ kann man aus dem Reaktionsgemisch die flüchtigen Anteile ohne Fraktionierung abdestillieren, wobei das Alkalichlorid sowie etwaiges überschüssiges Alkalialkoholat als Rückstand verbleiben, und den Methoxyessigsäureester durch Fraktionierung des Destillats gewinnen.

Die folgenden Beispiele sollen die Erfindung weiter erläutern, nicht aber ihren Umfang begrenzen, wie er in den Patentansprüchen definiert ist.

### Beispiele

### Beispiel 1 - Methoxyessigsäureisopropylester

434,1 g (4,0 mol) Chloressigsäuremethylester werden bei 25°C vorgelegt und gerührt. Man tropft 756,3 g (4,2 mol) 30%-ige Natriummethylatlösung innerhalb von 2,5 h so zu, daß eine Reaktionstemperatur von 65°C nicht überschritten wird. Nach der Zugabe läßt man noch 3 h bei Rückflußtemperatur weiter rühren. Dann wird aus dem Reaktionsgemisch durch Destillation 436,0 g Methanol abgetrennt. Zum Rückstand gibt man 480,8 g (8 mol) Isopropanol und erhitzt das Gemisch unter Rückfluß. Dabei wird über eine 0,5 m lange Multifil-Kolonne bei einem Rücklaufverhältnis von etwa 1:1 Methanol abgenommen (244,4 g Destillat). Nach 6,5 h beträgt der Umsatz des Methoxyessigsäuremethylesters zum Methoxyessigsäureisopropylester >98 %. Nach dem Abkühlen wird das Reaktionsgemisch am Rotationsverdampfer bei einer maximalen Badtemperatur von 120°C und einem Druck von <15 mbar zur Trockne eingeengt. Man erhält einen Rückstand von von 274,3 g und ein Destillat von 668,0 g. Das Destillat wird über eine 0,5 m lange Multifil-Kolonne fraktioniert. Man erhält als Hauptfraktion Methoxyessigsäureisopropylester (390,6 g. Ausbeute 74%) mit einer Reinheit von >99 %, einem Chlorgehalt von 10 ppm und einem Wassergehalt von <0,05 %.

### Beispiel 2 - Methoxyessigsäureisopropylester

434,1 g (4,0 mol) Chloressigsäuremethylester werden bei 25°C vorgelegt und gerührt. Man tropft 756,3 g (4,2 mol) 30%-ige Natriummethylatlösung innerhalb von 2,5 h so zu, daß eine Reaktionstemperatur von 65°C nicht überschritten wird. Nach der Zugabe läßt man noch 3 h bei Rückflußtemperatur weiter rühren. Dann wird aus dem Reaktionsgemisch durch Destillation 407,5 g Methanol abgetrennt. Zum Rückstand gibt man 480,8 g (8 mol) Isopropanol und erhitzt das Gemisch unter Rückfluß. Dabei wird über eine 0,5 m lange MultifilKolonne bei einem Rücklaufverhältnis von etwa 1:1 Methanol abgenommen (291,9 g Destillat). Nach 6,5 h beträgt der Umsatz des Methoxyessigsäuremethylesters zum Methoxyessigsäureisopropylester >98 %. Nach dem Abkühlen wird das Reaktionsgemisch abgesaugt und der Rückstand mit 450 g Isopropanol gewaschen. Man erhält einen Rückstand von von 412,1 g und eine flüssige Phase (Filtrat und Waschflüssigkeit) von 969,9 g. Die flüssige Phase wird über eine 0,5 m lange Multifil-Kolonne fraktioniert. Man erhält als Hauptfraktion Methoxyessigsäureisopropylester (405,0 g, Ausbeute 77%) mit einer organischen Reinheit von >99 %, einem Chlorgehalt von 10 ppm und einem Wassergehalt von <0,05 %.

### Beispiel 3 - Methoxyessigsäure-n-butylester

542,5 g (5,0 mol) Chloressigsäuremethylester werden bei 25°C vorgelegt und gerührt. Man tropft 945,4 g (5,25 mol) 30%-ige Natriummethylatlösung innerhalb von 2,5 h so zu, daß eine Reaktionstemperatur von 65°C nicht überschritten wird. Nach der Zugabe läßt man noch 3 h bei Rückflußtemperatur weiter rühren. Dann wird aus dem Reaktionsgemisch durch Destillation 518,8 g Methanol abgetrennt. Zum Rückstand gibt man 741,2 g (10 mol) n-Butanol und erhitzt das Gemisch unter Rückfluß. Dabei wird über eine 0,5 m lange Multifil-Kolonne bei einem Rücklaufverhältnis von etwa 1:1 Methanol abgenommen (398,7 g Destillat). Nach 6,5 h beträgt der Umsatz des Methoxyessigsäuremethylesters zum Methoxyessigsäure-n-butylester >98%. Nach dem Abkühlen wird das Reaktionsgemisch abgesaugt und der Rückstand mit 277 g n-Butanol gewaschen. Die flüssige Phase (Filtrat und Waschflüssigkeit) von 1.132,9 g. Die flüssige Phase wird über eine 0,5 m lange Multifil-Kolonne fraktioniert. Man erhält als Hauptfraktion Methoxyessigsäure-n-butylester (548,1 g, Ausbeute 75%) mit einer Reinheit von >99 %, einem Chlorgehalt von 10 ppm und einem Wassergehalt von <0,05 %.

### Beispiel 4 - Methoxyessigsäure-n-octylester

537,2 g (4,95 mol) Chloressigsäuremethylester werden bei 25°C vorgelegt und gerührt. Man tropft 936,4 g (5,2 mol) 30%-ige Natriummethylatlösung innerhalb von 2,5 h so zu, daß eine Reaktionstemperatur von 65°C nicht überschritten wird. Nach der Zugabe läßt man noch 3 h bei Rückflußtemperatur weiter rühren. Dann wird aus dem Reaktionsgemisch durch Destillation 511,9 g Methanol abgetrennt. Zum Rückstand gibt man 1,289,3 g (9,9 mol) n-Octanol und erhitzt das Gemisch unter Rückfluß. Dabei wird über eine 0,5 m lange Multifil-Kolonne bei einem Rücklaufverhältnis von etwa 1:1 Methanol abgenommen (303,5 g Destillat), wobei während der Abnahme die Sumpftemperatur bis auf 210°C anstieg. Nach 6,5 h beträgt der Umsatz des Methoxyessigsäuremethylesters zum Methoxyessigsäure-n-octylester >98 %. Das Reaktionsgemisch wird über eine 0,5 m lange Multifil-Kolonne fraktioniert. Zunächst werden n-Octanol und Methoxyessisäuremethylester abdestilliert. Dann erhält man als Hauptfraktion Methoxyessigsäure-n-octylester (740,0 g, Ausbeute 74%) mit einer Reinheit von >99 %, einem Chlorgehalt von 10 ppm und einem Wassergehalt von <0,05 %.

## Patentansprüche

1. Verfahren zur Herstellung von Methoxyessigsäureestern, dadurch gekennzeichnet, daß ein Chloressigsäureester mit einem Alkalimethylat zum Methoxyessigsäureester umgesetzt wird.

2. Verfahren nach Anspruch 1, dadurch gekennzeichnet, daß der Chloressigsäureester sich von einem Alkanol mit 1 bis 6 Kohlenstoffatomen oder einem Alkoxyalkohol mit 3 bis 6 Kohlenstoffatomen ableitet.

3. Verfahren nach einem der Ansprüche 1 oder 2, dadurch gekennzeichnet, daß das Alkalimethylat Kalium- oder Natriummethylat ist und in einer Menge von 1 bis 2 Äquivalenten angewandt wird.

4. Verfahren nach Anspruch 3, dadurch gekennzeichnet, daß die Menge des Alkalimethylats 1 bis 1.5 Äquivalente beträgt.

5. Verfahren nach Anspruch 3, dadurch gekennzeichnet, daß die Menge des Alkalimethylats 1 bis 1.2 Äquivalente beträgt.

6. Verfahren nach einem der Ansprüche 1 bis 5, dadurch gekennzeichnet, daß die Umsetzung bei 20 bis 100°C stattfindet

7. Verfahren nach einem der Ansprüche 1 bis 5, dadurch gekennzeichnet, daß die Umsetzung bei 60 bis 80°C stattfindet

8. Verfahren nach einem der Ansprüche 1 bis 7, dadurch gekennzeichnet, daß der Methoxyessigsäureester nach seiner Isolierung aus dem Reaktionsgemisch oder direkt im Reaktionsgemisch mit einem höheren Alkohol zu einem Methoxyessigsäureester des höheren Alkohols umgesetzt wird.

9. Verfahren nach Anspruch 8, dadurch gekennzeichnet, daß der höhere Alkohol ein Alkanol mit 2 bis 10 Kohlenstoffatomen oder ein Alkoxyalkanol mit 4 bis 10 Kohlenstoffatomen ist.

10. Verfahren nach Anspruch 8 oder 9, dadurch gekennzeichnet, daß die Umesterung bei einer Temperatur von 80 bis 250°C durchgeführt wird.

11. Verfahren nach einem der Ansprüche 8 bis 10, dadurch gekennzeichnet, daß man als Katalysator überschüssiges Alkalimethylat, ein Alkoholat eines Alkohols mit 1 bis 8 Kohlenstoffatomen, einen Lewis-sauren Katalysator oder eine Broenstedt-Säure verwendet.

12. Verfahren nach einem der Ansprüche 1 bis 11, dadurch gekennzeichnet, daß man die Umsetzung bei einem Druck von 0,8 bis 3 bar durchführt.

13. Verfahren nach einem der Ansprüche 1 bis 11, dadurch gekennzeichnet, daß man die Umsetzung bei einem Druck von 0,5 bis 1,5 bar durchführt.

14. Verfahren nach einem der Ansprüche 1 bis 13, dadurch gekennzeichnet, daß man aus dem Reaktionsgemisch das Alkalichlorid abtrennt und den Methoxyessigsäureester durch Fraktionieren der flüssigen Phase gewinnt, wobei etwaiges überschüssiges Alkalialkoholat im Rückstand verbleibt.

15. Verfahren nach einem der Ansprüche 1 bis 13, dadurch gekennzeichnet, daß man aus dem Reaktionsgemisch die flüchtigen Anteile ohne Fraktionierung abdestilliert, wobei das Alkalichlorid sowie etwaiges überschüssiges Alkalialkoholat als Rückstand verbleiben, und den Methoxyessigsäureester durch Fraktionierung des Destillats gewinnt.
